# EUROPÄISCHE PATENTSCHRIFT

(11) **EP 4 543 835 B1**
(45) Veröffentlichungstag und Bekanntmachung des Hinweises auf die Patenterteilung: **29.04.2026**
(21) Anmeldenummer: 23737876.5
(22) Anmeldetag: 22.06.2023
(51) Int. Cl.: C07C 31/04, C07C 29/151, C25B 1/04, C25B 15/08, C01B 3/34

(54) **METHANOLHERSTELLUNG AUS BIOMASSE UND GRÜNEM WASSERSTOFF**
METHANOL PRODUCTION FROM BIOMASS AND GREEN HYDROGEN
PRODUCTION DE MÉTHANOL À PARTIR DE BIOMASSE ET D'HYDROGÈNE VERT

(30) Priorität: 27.06.2022 DE 102022115977
(43) Veröffentlichungstag der Anmeldung: 30.04.2025
(73) Patentinhaber: Fev Group GmbH, 52078 Aachen (DE)
(72) Erfinder: SCHNORBUS, Thorsten, 59955 Winterberg (DE)
(74) Vertreter: Brandt, Maximilian
(86) Internationale Anmeldenummer: PCT/DE2023/100473
(87) Internationale Veröffentlichungsnummer: WO 2024/002419

(56) Entgegenhaltungen:
- EP-B1- 3 452 438

## Beschreibung

Die vorliegende Erfindung betrifft ein Verfahren zur Methanolsynthese und ein entsprechendes System.

Aus EP 3 452 438 B1 ist ein Verfahren zur Herstellung von Bio-Methanol bekannt, bei dem elektrolytisch gewonnener Wasserstoff und organische Abfallstoffe zugeführt werden. Ferner ist aus EP 3 017 015 B1 bekannt, in einem ersten Schritt der Aufbereitung von Biomasse Steamcracker zu verwenden, um einen kohlenwasserstoffhaltigen Einsatzstoff in kürzere Ketten zu zerteilen. Derartige Verfahren und Vorrichtungen sind darauf fokussiert, aus bereitgestellten Eingangsstoffen, wie insbesondere Biomasse und Wasserstoff, die chemischen Umwandlungen möglichst effektiv durchzuführen. Es wird hier typischerweise nicht betrachtet, dass in manchen Fällen die Einsatzstoffe für die Umwandlungen nicht ausreichend vorhanden sein könnten und/oder dass es zu Schwankungen in der mengenmäßigen bzw. zeitlichen Bereitstellung der Einsatzstoffe kommen kann. Weiterhin soll der Bedarf von nicht regenerativ gewonnenem Strom für das Gesamtsystem der Methanolsynthese reduziert oder vermieden werden.

Die vorliegende Erfindung zielt auf dieses Problem. Die Merkmale der unabhängigen Ansprüche beschreiben erfinderische Vorteile. Bevorzugte Weiterbildungen sind Gegenstand der abhängigen Ansprüche.

Bei einem Verfahren zur Herstellung von Methanol wird ein Synthesegas aus Biomasse gewonnen und einer Methanolsynthesevorrichtung zugeführt. Dabei wird in einer Hauptbetriebsart, bei der ausreichend elektrischer Strom für eine elektrolytische Wasserstoff-Gewinnung verfügbar ist, entsprechend elektrolytisch gewonnener Wasserstoff der Methanolsynthesevorrichtung zugeführt. Und in einer Nebenbetriebsart, bei der kein ausreichender elektrischer Strom für die elektrolytische Herstellung von Wasserstoff verfügbar ist, wird ein Restgas, dass sich bei der Abtrennung des Synthesegases von einem aus der Biomasse gewonnenem Biogas ergibt, einem Generator zugeführt, um elektrischen Strom für an dem Verfahren beteiligte Vorrichtungen herzustellen. Das Restgas ist also die Differenz von dem erzeugten Biogas und dem Synthesegas, welches insbesondere so abgetrennt wurde, dass es unmittelbar in der Methanolsynthesevorrichtung einsetzbar ist. In der Nebenbetriebsart wird insbesondere keine Elektrolyse betrieben. Mit dem Begriff "Strom für eine elektrolytische Wasserstoffgewinnung" ist insbesondere regenerativ erzeugter Strom, wie bspw. aus Fotovoltaik oder Windkraft gemeint. Grundsätzlich sind aber andere Stromquellen, wie z.B. auch aus Atomkraft mit umfasst.

Standardmäßig ist eine Methanolsynthesevorrichtung eine Einheit, die Synthesegas (H2 und CO) in Methanol umsetzt. In diesem besonderen Fall wird allerdings anstelle CO überwiegend CO2 dem Prozess zugeführt. Die Synthese-Einheit arbeitet mit einer Rezirkulationspumpe/gebläse, welches die nicht konvertierten Ausgangsstoffe erneut der Synthese zuführt. Bei der Synthese entstehen Methanol und Wasser. Da bei den Feed-Gasen auch immer kleine Mengen an Schad-/Fremd- oder Inertgasen dem Prozess zugeführt werden, muss durch einen Purge-Gas-Strom eine Anreicherung dieser Gase vermieden werden, um die Effizienz des Syntheseprozesses nicht zu beeinträchtigen. Der Prozess ist exotherm und die entstehende Wärme kann in anderen Teilen der Anlage verwendet werden (zum Vorheizen der Destillation oder des HyGas-Prozesses). Die ausgehenden Gase des Purge-Gas-Stroms können von diversen Komponenten des Systems genutzt werden, wie insbesondere den Generator oder den Oxyfuel-Brenner.

Einerseits lässt sich Menge von Biogas in einem Bioreaktor, insbesondere in einem HyGas-Erzeuger, zeitlich nicht gut variieren, so dass sich ein zeitlich weitgehend konstanter Biogasstrom ergibt. Andererseits schwankt die Menge von Fotovoltaik-Strom tageszeitabhängig, so dass es dadurch zu Schwankungen im (tages-)zeitlichen Verhältnis dieser Eingangsstoffe für die Methanolsynthese kommt. Aufgrund der beiden vorstehend beschriebenen Betriebsarten des erfinderischen Verfahrens (verbunden mit Zwischenstufen) gelingt es zu jedem Zeitpunkt eine maximal mögliche Methanolmenge zu erhalten und gleichzeitig die benötigte Menge an nicht-grünem Strom insgesamt zu verringern. Zwar ist die Menge des erzeugten Methanols in der Nebenbetriebsart, also bei einem Mangel an grünem Strom, im Vergleich zur Hauptbetriebsart, deutlich reduziert, aber durch die Zuführung des Restgases aus der Biomassenverwertung an den Generator, wird es dennoch möglich, die Synthese durchzuführen, ohne auf nicht-grünen Strom zurückgreifen zu müssen.

Vorteilhaft ist, wenn zumindest in der Hauptbetriebsart und bevorzugt auch in der Nebenbetriebsart, ein Restgas, welches sich bei der Abtrennung des Synthesegases von einem aus Biomasse erzeugten Biogases ergibt, einer Aufbereitung zugeführt wird, um hierüber weiteres Synthesegas zu erzeugen, welches ebenfalls der Methanolsynthesevorrichtung zugeführt wird. Die Methanolsynthesevorrichtung benötigt einfache chemische Verbindungen, wie z.B: H₂, CO₂, welche zunächst in einem ersten Schritt von dem Biogas abgetrennt werden. Um eine hohe Ausbeute von Synthesegas aus dem Biogas zu erhalten, werden in dem beschriebenen zweiten Schritt die restlichen Bestandteile, also das Restgas, derart umgewandelt, dass es als ein "weiteres Synthesegas" verwendbar ist.

Bei der Aufbereitung ist es insbesondere vorteilhaft, wenn das Restgas in einem bestimmten Verhältnis einem Oxyfuel-Brenner und einem Reformer zugeführt wird, wobei im Oxyfuel-Brenner eine Oxidation durchgeführt und das oxidierte Gas dem Reformer zugeführt wird und der Reformer mit diesen Gasen eine Verarbeitung, insbesondere eine Reduktion, durchführt, um sie als das weitere Synthesegas der Methanolsynthesevorrichtung zuführen zu können. Auch in der Nebenbetriebsart kann diese Aufbereitung zum Einsatz kommen. Ein Oxyfuelbrenner ist insbesondere eine Brennereinheit, die Kohlenwasserstoffe und andere Gase verbrennt, um Wärme zu erzeugen. In diesem besonderen Fall wird das Gas mit reinem Sauerstoff verbrannt, um die Einbringung von z.B. Stickstoff aus der Luft zu vermeiden. Das Verbrennungsluftverhältnis ist unterstöchiometrisch, um einen sauerstofffreien Abgasstrom zu erzeugen, der seinerseits in den weiteren Prozessschritten als Edukt genutzt werden kann. Ein Reformer ist also eine chemische Einheit, die Kohlenwasserstoffe in Wasserstoff und Kohlendioxid/Kohlenmonoxid umsetzt. Der Prozess ist endotherm und muss so von außen mit Wärme versorgt werden.

Ferner ist vorteilhaft, wenn abhängig von dem verfügbaren elektrischen grünen Strom oder abhängig vom verfügbaren Wasserstoff zwischen der Haupt- und Nebenbetriebsart umgeschaltet werden kann (das System ist derart ausgerichtet, dass es diese Umschaltung vornehmen kann), oder Mischformen der Betriebsarten zum Einsatz kommen, wobei die Mischformen sich bspw. in dem Verhältnis des Restgases unterscheiden, das entweder dem Generator oder der Aufbereitung zugeführt wird. Als Kriterium für das Umschalten (bzw. des anteilmäßigen Einstellens oder Regelns) eignet sich besonders die Menge des verfügbaren grünen Stroms, da dieser einfach zu messen ist. Die Menge des elektrolytisch gewonnenen Wasserstoffs ist proportional zu dem grünen Strom, so dass auch dieser Elektrolyse-Wasserstoff als Kriterium verwendbar ist. Letztendlich ist aber der gesamte in dem System der Methanolsynthesevorrichtung vorhandene Wasserstoff ein wesentliches Kriterium für die Steuerung/Regelung zwischen beiden Betriebsarten, wobei diese auch abhänigig vom Synthesegas ist, welches je nach Grundstoffqualität variieren kann. Und da dieser auch durch den Wasserstoffanteil im Synthesegas (bzw. den Synthesegasen) beeinflusst wird, kann alternativ auch diese Größe als Kriterium für die Umschaltung herangezogen werden.

Zusätzlich ist dabei vorteilhaft, wenn die Umschaltung zwischen den Betriebsarten oder das Einstellen des Verhältnisses der Betriebsarten automatisiert in Abhängigkeit von dem für die Elektrolyse verfügbaren Stroms oder der Menge des verfügbaren Wasserstoffs, sowie des C/H Verhältnisses des Synthese-/Bio-Gases stattfindet, so dass so ein autonom arbeitendes System entsteht, bei dem keine Kontrollperson regelmäßig eingreifen muss. Somit ist das System bevorzugt derart eingerichtet, dass es in Abhängigkeit von dem für die Elektrolyse verfügbaren Stroms oder der Menge des verfügbaren Wasserstoffs zwischen den Betriebsarten (vorzugsweise automatisiert) umschalten kann.

Vorteilhaft ist ferner, wenn in der Hauptbetriebsart und der Nebenbetriebsart aus Biomasse ein Biogas erzeugt und in einem Hydrogenabtrenner aus dem Biogas das Synthesegas abgetrennt wird, welches Wasserstoff H2 und insbesondere auch Kohlendioxid CO2 umfasst. Dies beschreibt beispielhaft, wie das Synthesegas aus der Biomasse gewonnen werden kann. Ein Hydrogenabtrenner umfasst im Wesentlichen eine Membraneinheit, die Wasserstoff (und ggf. Wasserstoff und CO2) aus einem Gasstrom abtrennt. Hier sind neben Membranlösungen aber auch andere Technologien, wie z.B. Druckwechseladsorption oder ähnliches denkbar.

Zudem wird vorteilhafterweise betrachtet, dass Prozesswärme, die von dem Generator und/oder dem Oxyfuel-Brenner, sowie der Methanolsynthese erzeugt wird, an einen HyGas-Erzeuger zur Erzeugung des Biogases und/oder an den Reformer und/oder an die Methanoldestillation bzw. -synthese geleitet wird. Dies ist vorteilhaft, da die Komponenten, die Wärme benötigen, auf diese Weise unmittelbar mit der entstandenen Prozesswärme versorgt werden.

In einer Weiterbildung wird ein Speicher für Sauerstoff bereitgestellt, wobei in der Hauptbetriebsart der Speicher mit elektrolytisch gewonnenem Sauerstoff befüllt wird und der Sauerstoff wird in der Nebenbetriebsart zum Betrieb des Oxyfuel-Brenners verwendet. Der Oxyfuel-Brenner muss i.d.R. mit reinem Sauerstoff betrieben werden. Hierdurch kann auch während des Nebenbetriebs, also zu Phasen ohne Generation von Elektrolysesauerstoff, der Oxyfuel-Brenner effektiv eingesetzt werden und so ergeben sich 24-Stunden Zyklen, über die der Sauerstoff iterativ gespeichert und für den Nebenbetrieb bereitgestellt werden kann.

In einer Weiterbildung wird ein Speicher für CO2 bereitgestellt, wobei in der Nebenbetriebsart der Speicher mit aus dem Synthesegas gewonnenem CO2 befüllt werden kann und das gespeicherte Kohlenstoffdioxid in der Hauptbetriebsart zur Methanolsynthese genutzt werden kann. Hierdurch kann in Phasen mit hoher Verfügbarkeit von grünem Strom (z.B. bei intensiver Sonneneinstrahlung / hohem Windaufkommen) während des Hauptbetriebs eine erhöhte Produktion von Wasserstoff umgesetzt werden. Auch hiermit ergeben sich 24-Stunden Zyklen, über die der Kohlenstoffdioxid iterativ gespeichert und für den Hauptbetrieb bereitgestellt werden kann.

In einer alternativen oder zusätzlichen Weiterbildung kann ein Teil des grünen Stromes dazu eingesetzt werden, mithilfe einer Abscheidevorrichtung zusätzliches CO2 durch Abscheidung aus der Umgebungsluft (Direct Air Capture - DAC) zu erzeugen und dem Synthesegas hinzuzufügen. Dadurch kann in H2-Überschussphasen, also Phasen mit hoher Verfügbarkeit von grünem Strom, eine erhöhte Ausbeute von Methanol realisiert werden.

Zusätzlich kann in einer Weiterbildung ein H2-Speicher bereitgestellt werden, um auch im Nebenbetrieb weitere Flexibilität in der H2 Zufuhr zur Methanol Synthese zu generieren.

Auch wird hier ein System zur Herstellung von Methanol betrachtet, mit einem HyGas-Erzeuger zur Speisung eines Hydrogenabtrenner, der eingerichtet ist, vor allem Wasserstoff H₂ abzuscheiden, welches als Synthesegas einer Methanolsynthesevorrichtung zugeführt wird, wobei verbleibende Anteile aus dem Hydrogenabtrenner als ein Restgas einem Oxyfuel-Brenner und/oder einem Reformer und/oder einem Generator zugeführt werden, wobei das System in einer Hauptbetriebsart eingerichtet ist, der Methanolsynthesevorrichtung Elektrolyse-Wasserstoff zuzuführen. Und in einer Nebenbetriebsart findet keine Elektrolyse statt. Ferner ist eine Steuerung bzw. Regelung vorgesehen, um bei einer Reduktion des zugeführten Elektrolyse-Wasserstoffs einen Restgasstrom von dem Hydrogenabtrenner an einen Generator zu starten oder zu erhöhen, indem ein Restgasstrom von dem Hydrogenabtrenner an den Reformer und insbesondere auch den Oxyfuelbrenner entsprechend reduziert wird. Der Elektrolyse-Wasserstoffs wird bevorzugt durch Elektrolyse mit Strom gewonnen, der durch Solar- oder Windenergie erzeugt wurde. Der Kohlenwasserstoffstrom von dem Wasserstoffabtrenner zu sowohl dem Oyxfuel-Brenner als auch dem Reformer findet in einem vorgegebenen Verhältnis statt, welches so eingestellt bzw. geregelt wird, dass von dem Reformer ein möglichst reines H₂-, CO- und CO₂-Gemisch an die Methanolsynthesevorrichtung geleitet wird, welches zudem stöchiometrisch für die Synthese geeignet ist. Das System ist eingerichtet, die anspruchsgemäßen Verfahren auszuführen.

Nachfolgend wird die Erfindung beispielhaft beschrieben. Es zeigen
- Fig 1:: einen Ablaufplan des Systems in einem ersten Ausführungsbeispiel der Hauptbetriebsart, wobei dieser Plan auch bei Mischformen der Betriebsarten verwendet wird,
- Fig 2:: einen Ablaufplan des Systems in einem ersten Ausführungsbeispiel einer Nebenbetriebsart,
- Fig 3:: einen Ablaufplan des Systems in einem zweiten Ausführungsbeispiel der Hauptbetriebsart, wobei dieser Plan auch bei Mischformen der Betriebsarten verwendet wird und
- Fig 4:: einen Ablaufplan des Systems in einem zweiten Ausführungsbeispiel der Nebenbetriebsart.

In der nachfolgenden Beschreibung der bevorzugten Ausführungsformen eines Systems zur Methanolsynthese werden eine Haupt- und eine Nebenbetriebsart beschrieben, welche sich insbesondere dadurch unterscheiden, ob der Vorrichtung ausreichend grüner Strom zum Betrieb von einer Elektrolyse-Vorrichtung zugeführt wird, und deren einzelnen Komponenten selbst. Grüner Strom ist insbesondere Strom, der durch Photovoltaik oder Windkraft gewonnen wird. Da Photovoltaik nur tagsüber arbeitet, kann die Hauptbetriebsart alternativ auch als "Tagbetrieb" und die Nebenbetriebsart folglich als "Nachtbetrieb" bezeichnet werden.

Grundsätzlich werden also zwei Betriebsarten der Vorrichtung unterschieden. Nämlich, dass in der Hauptbetriebsart ausreichend (regenerativ erzeugter) Strom zugeführt wird, um eine maximale Methanolsynthese zu ermöglichen. In dieser Betriebsart wird grundsätzlich diese maximale Methanolsynthese durch den Kohlen- und/oder Sauerstoffanteil in einem nachfolgend beschriebenen Biogas 15 begrenzt. In der Nebenbetriebsart wird der Anlage kein durch regenerative Techniken erzeugter Strom zugeführt. Da bevorzugt kein nicht-regenerativer Strom verwendet werden soll, wird das System in der Nebenbetriebsart derart gedrosselt, dass die Methanolproduktion reduziert wird und stattdessen erzeugt ein wesentlicher Anteil des Biogases 15 in einem Generator 50 Strom, für den Betrieb der einzelnen Komponenten des Systems. Der Begriff "Generator" steht als ein Überbegriff für eine Vielzahl von technischen Möglichkeiten aus organischen Substanzen Strom zu gewinnen. Insbesondere können die organischen Substanzen in einer Verbrennungskammer verbrannt werden, wodurch eine Flüssigkeit verdampft und damit in einer Turbine der gewünschte Strom erzeugt wird.

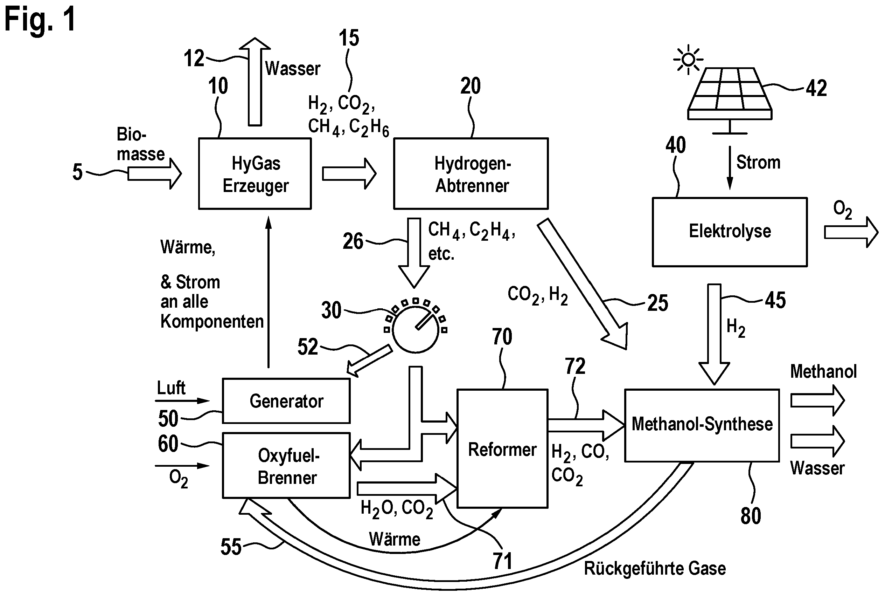

Oben links wird in dem Diagramm der Fig. 1 einem HyGas-Erzeuger 10 Biomasse 5 zugeführt. Der Begriff "Biomasse" wird als ein Oberbegriff verstanden und umfasst bspw. Klärschlamm, aber insbesondere auch organische Abfälle, wie zum Beispiel Bio-Abfall wie Grünschnitt oder Trester, Gärreste aus Biogas-Anlagen und Gülle, aber auch Abfälle aus der Lebensmittelindustrie. Insbesondere aufgrund der Klärschlammverordnung ist es nicht mehr zulässig, Klärschlamm zu verbrennen. Vielmehr ist es sogar sinnvoll, die enthaltenen (oder gewinnbaren) Grundstoffe möglichst mehrwertschaffend zu veredeln, so wie dies bei der nachfolgend beschriebenen Methanolgewinnung geschieht. Als HyGas-Erzeuger 10 werden insbesondere Vorrichtungen bezeichnet, die eine nasse organische Masse mit überkritischem Wasser, bei einem Druck von mehr als 250 bar und einer Temperatur von über 600°C behandeln. Dabei wird Biogas 15 abgespalten, bei dem es sich um ein Gas mit hohem Methan- CH₄- und Wasserstoff-H₂-Gehalt handelt. Ferner sind weitere (bevorzugt kurzkettige) Kohlenwasserstoffe enthalten, wie insbesondere C₂ H₆. aber auch CO, CO₂. Als ein Nebenprodukt erzeugt der HyGas-Erzeuger 10 Wasser 12, welches hier neben einer partiellen Nutzung in der Elektrolyse aber nicht näher betrachtet wird. Insbesondere oder alternativ kann der HyGas-Erzeuger 10 ein anaerober Fermenter sein.

Das erzeugte Biogas 15 wird einem Hydrogen-Abtrenner 20 zugeführt. Dort werden Bestandteile, die unmittelbar in der Methanolsynthesevorrichtung 80 verwendbar sind, abgetrennt und als Synthesegas 25 der Methanolsynthesevorrichtung 80 zugeführt. Bei dem Synthesegas 25 handelt es sich vor allem Wasserstoff H₂. Aus technischen Gründen kann auch ein nennenswerter Anteil von Kohlendioxid CO₂ enthalten sein. Und ggf. können in gewissen kleineren Mengen auch andere Gase enthalten sein.

Die Anteile des Biogases 15, die nicht als Synthesegas 25 weitergeleitet werden können, werden nachfolgend als Restgas 26 bezeichnet und an einen Regler oder Schalter 30 geleitet. Dieser wird in der Folge vereinfachend als "Regler" bezeichnet. Im Sinne der Regelungstechnik kann dies entweder eine Steuerung oder ein Regelkreis sein. Es ist insbesondere dann eine Steuerung, wenn der verfügbare grüne Strom als Stellgröße herangezogen wird. Wenn hingegen die Gesamtmenge des Wasserstoffs, der der Methanolsynthesevorrichtung 80 zugeführt wird, als Stellgröße herangezogen wird, so kann es sich um eine Regelung handeln, da abhängig von der Regelstellung sich die Wasserstoffmenge ändert, die über einen Reformer 70 der Methanolsynthesevorrichtung 80 zugeführt wird. Der Regler 30 teilt den zugeführten Restgas(massen)strom 26 in zwei Teil(massen)ströme auf, wobei die Aufteilung in den Betriebsweisen unterschiedlich ist.

In der Hauptbetriebsweise umfasst das System eine Elektrolysevorrichtung 40, die bevorzugt mit regenerativ erzeugtem Strom (Photovoltaik, Windkraft) versorgt wird und in der Wasser in Elektrolysewasserstoff 45 und Elektrolysesauerstoff aufgespalten wird. Der Elektrolysewasserstoff 45 wird unmittelbar zu der Methanolsynthesevorrichtung 80 geleitet. Der Elektrolysesauerstoff wird bedarfsgerecht einem Oxyfuel-Brenner 60 zugeführt oder aus dem System abgeleitet. Die Methanolsynthesevorrichtung 80 produziert das gewünschte Biomethanol und Wasser als ein Abfallprodukt. Ferner verbleiben Restgase 55, die hier nicht verwendet werden können und die in einer Rückführung einem Generator 50 zugeführt werden. Der Generator 50 verbrennt die ihm zugeführten Gase und erzeugt hierüber Strom und Wärme, die ihrerseits jeweils an unterschiedliche Komponenten des Systems zugeführt werden, bei denen ein entsprechender Bedarf besteht.

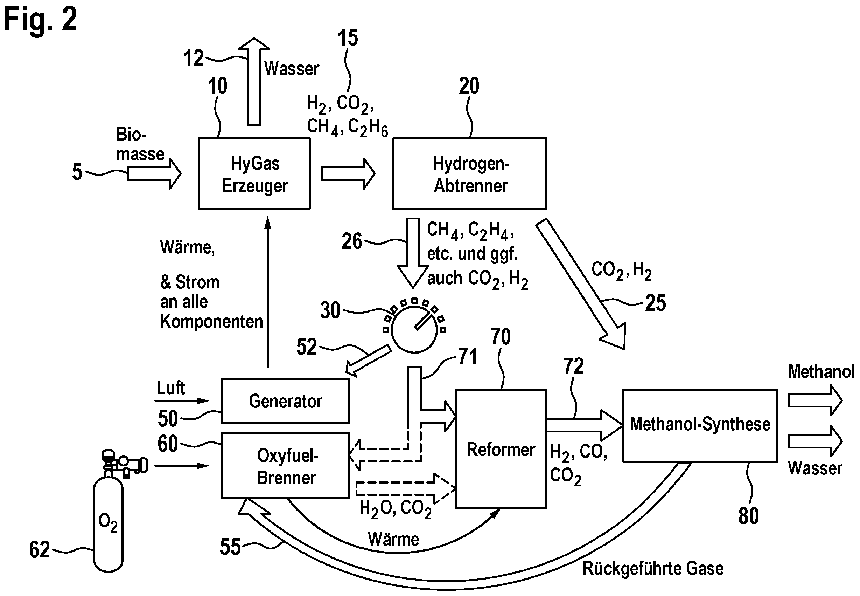

Wie bereits erwähnt, wird in dem Regler 30 das zugeführte Restgas 26 entsprechend dessen Regelstellung in einem bestimmten Verhältnis in Teil(massen)ströme aufgeteilt. Die Breite der Pfeile der Fig. 1 und 2 drückt schematisch das Aufteilungsverhältnis bei der Haupt- bzw. Nebenbetriebsart aus. Entsprechend geht bei der Hauptbetriebsart der Fig. 1 ein größerer Anteil (insbesondere optional komplett zu 100%) in eine Aufbereitung, die einen Oxyfuel-Brenner 60 und einen Reformer 70 umfasst. Von der Aufbereitung 60, 70 führt ein entsprechender Massestrom als ein weiteres Synthesegas 72 zu der Methanol-Synthese 80. Das Restgas 26, das der Aufbereitung 60, 70 zugeleitet wird, wird innerhalb der Aufbereitung 60, 70 zwischen dem Oxyfluel-Brenner 60 und dem Reformer 70 in einem bestimmten Verhältnis aufgeteilt. Dieses Verhältnis wird so bestimmt, dass in der Methanolsynthesevorrichtung 80 eine stöchiometrisch bestmögliche Synthese des Methanols ermöglicht wird. Die Bestimmung dieses Verhältnisses kann über einen Regelkreis (nicht dargestellt) automatisiert erfolgen.

Der Oxyfuel-Brenner 60 wird mit reinem Sauerstoff, aber unterstöchiometrisch, betrieben und dessen Ausgangprodukte sind insbesondere Wasser und CO₂, die zu dem Reformer 70 geleitet werden. Zudem erhält der Reformer 70 einen Anteil des Restgases 26 und in ihm finden Umwandlungsprozesse statt. Diese endothermen Prozesse werden mit Wärme des Oxyfuel-Brenners 60 und/oder des Generators 50 versorgt. Die Produktgase des Reformers 70 sind H₂, CO und CO₂ und werden als weiteres Synthesegas 72 der Methanol-Synthese 80 zugeführt.

In Fig. 2 ist die Nebenbetriebsart gezeigt, bei der kein Wasserstoff aus einer Elektrolyse erzeugt und der Methanolsynthesevorrichtung 80 bereitgestellt wird. Da dadurch die Produktion des Methanols in der Methanolsynthesevorrichtung 80 reduziert ist, sinkt auch der Bedarf der Synthesegase 25 und 72, wobei insbesondere der CO₂-Bedarf reduziert ist. Somit wird insbesondere ein Teil des im Hydrogenabtrenner 20 abgetrennten (oder abtrennbaren) Synthesegases 25 nicht der Methanolsynthesevorrichtung 80 zugeführt, sondern in den Strom des Restgases 26 eingeleitet. Anders ausgedrückt wird bevorzugt vom Biogas 15 lediglich ein reduzierter Strom des Synthesegases 25 abgetrennt. Der jeweilige Anteil ergibt sich aus dem Bedarf der Methanolsynthesevorrichtung 80.

Von dem ggf. so veränderten Restgas 26 wird in der Nebenbetriebsart ein größerer Anteil als Generatorgas 52 an den Generator 50 geleitet, wie es durch die Breite des Pfeils der Fig. 2 angedeutet wird. Dort wird es verbrannt, um hierüber elektrischen Strom und Wärme zu erzeugen, welcher in den Komponenten des Systems, die einen entsprechenden Bedarf haben, gebraucht wird. Hierdurch kann der Bedarf von nicht regenerativ gewonnenem Strom für das Gesamtsystem der Methanolsynthese reduziert oder vermieden werden. Die dabei entstehende Wärme wird auch, vergleichbar mit dem Oxyfuel-Brenner 60, an die entsprechenden Komponenten des Systems geleitet.

Abhängig von den konkreten Gaszusammensetzungen kann es möglich sein, dass der Anteil des Restgases 26, der aufbereitet werden soll, lediglich an den Reformer 70 geleitet wird, so dass der Oxyfuel-Brenner 60 in diesem Fall nicht benötigt wird. Auch kann alternativ der Anteil, der an die Aufbereitung 60, 70 geleitet wird auf Null gesetzt werden. Letzteres tritt dann auf, wenn der Volumenstrom des Synthesegas 25 für die Methanolsynthesevorrichtung 80 ausreicht. Anteile des Synthesegases 25, die für die Synthese nicht verwendet werden, werden als rückgeführte Gase 55 dem Generator 50 zugeführt und thermisch verwertet.

Der Regler 30 kann also das Restgas 26 zu 100% dem Generator 50 zuführen. Andererseits gibt es in der Nebenbetriebsart nicht den Zustand, dass das Restgas 26 zu 100% der Aufbereitung 60, 70 zugeführt wird. Dieser Zustand, also dass der Generator 50 nicht mit Restgas 26 versorgt wird, ist allenfalls in der Hauptbetriebsart sinnvoll. Auch in der Hauptbetriebsart kommt der Generator 50 dennoch stets zum Einsatz, da in ihm die rückgeführten Gase 55 thermisch verwertet werden. Auch Zwischenstufen sind (bevorzugt stufenlos) einstellbar. Alternativ zu Zwischenstufen ist auch ein jeweiliges komplettes Umschalten der Betriebsarten möglich.

Auch kann optional ein Sauerstoffspeicher 62 zum Einsatz kommen. Wenn das System tagsüber mit Photovoltaikstrom in der Hauptbetriebsart betrieben wird, fällt typischerweise ein Überschuss von Elektrolyse-Sauerstoff an. Dieser Überschuss ergibt sich als die Differenz von dem elektrolytisch gewonnenen Sauerstoff mit dem Sauerstoffbedarf des Oxyfuel-Brenners 60. Dieser Überschuss kann in dem Sauerstoffspeicher 62 für die Nebenbetriebsart während der Nacht gespeichert werden, so dass auch in der Nacht der Oxyfuel-Brenner 60 arbeiten kann. Der Oxyfuel-Brenner 60 muss nämlich mit reinem Sauerstoff betrieben werden, um seine Funktion zu gewährleisten und um sicherzustellen, dass keine Fremdgase, wie z.B. Stickstoff, der Methanolsynthesevorrichtung 80 zugeführt werden.

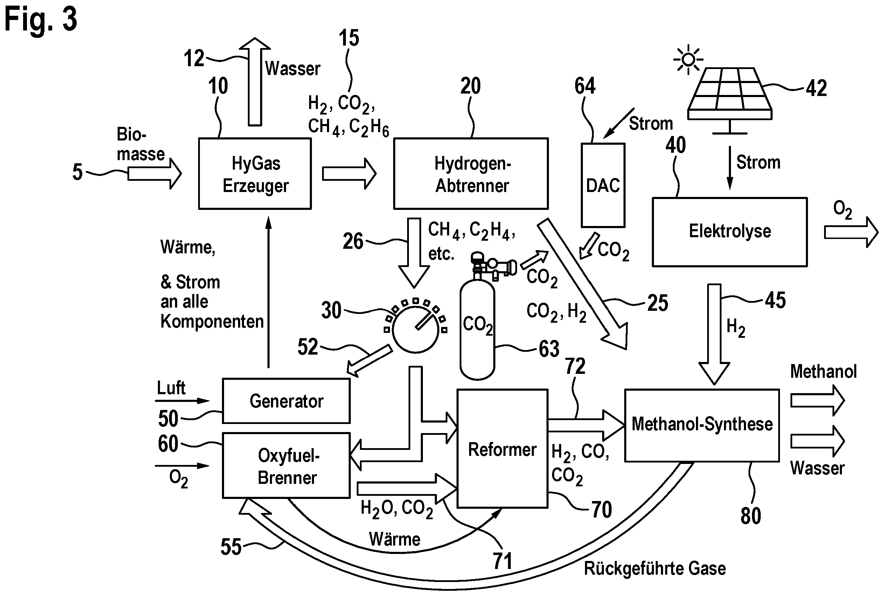

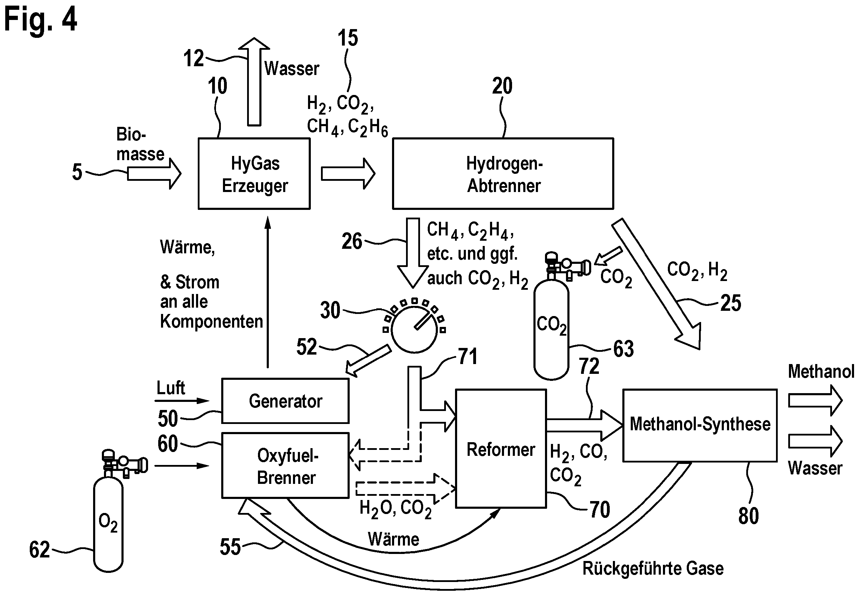

Figuren 3 und 4 zeigen jeweils einen zweites Ausführungsbeispiel eines Ablaufplans der Haupt- und Nebenbetriebsart. Dieses Ausführungsbeispiel unterscheidet sich nur in einem Aspekt von dem in den Figuren 1 und 2 dargestellten Ablaufplan, wobei im Folgenden nur auf diesen Aspekt eingegangen wird. Gemäß des weiteren Ausführungsbeispiels des Ablaufplans wird bei hoher Verfügbarkeit des grünen Stromes, also z.B. bei intensiver Sonneneinstrahlung, ein Teil des Stromes in einer Abscheidevorrichtung 64 dazu verwendet, Kohlenstoffdioxid aus der Umgebungsluft auszuscheiden (Direct Air Capture) und dem Synthesegas 25 hinzuzufügen. Hierdurch kann in Phasen überschüssigen grünen Stromes verhindert werden, dass in der Methanolsynthesevorrichtung 80 ein Überschuss an Wasserstoff entsteht. Stattdessen kann der überschüssige grüne Strom dazu genutzt werden, weiteres Kohlenstoffdioxid zu erzeugen, um die Methanolausbeute zu maximieren.

Alternativ oder zusätzlich hierzu kann auch ein CO2-Speicher 63 eingesetzt werden, welcher im Nebenbetrieb mit überschüssigem Kohlenstoffdioxid befüllt wird, welches während des Hauptbetriebs, in Phasen überschüssigen grünen Stromes, wieder dem Synthesegas 25 zugegeben werden kann. Hierbei steuert der Regler 30 die Befüllung und Entleerung des CO2-Speichers 63 und die Zuführung grünen Stromes zur Abscheidevorrichtung 64.

## Patentansprüche

1. Verfahren zur Herstellung von Methanol,
bei dem ein Synthesegas (25), das aus Biomasse (5) gewonnen wurde, einer Methanolsynthesevorrichtung (80) zugeführt wird,
wobei
in einer Hauptbetriebsart, bei der ausreichend elektrischer Strom für eine elektrolytische Wasserstoff-Gewinnung verfügbar ist, entsprechend elektrolytisch gewonnener Wasserstoff (45) der Methanolsynthesevorrichtung (80) zugeführt wird,
und wobei in einer Nebenbetriebsart, bei der kein ausreichender elektrischer Strom für die elektrolytische Herstellung von Wasserstoff (45) verfügbar ist, ein Restgas (26), das sich bei der Abtrennung des Synthesegases (25) von einem aus der Biomasse (5) gewonnenem Biogas (15) ergibt, einem Generator (50) zugeführt wird, um elektrischen Strom für an dem Verfahren beteiligte Vorrichtungen (10, 20, 30, 40, 70, 80) bereitzustellen.

2. Verfahren gemäß Anspruch 1, wobei zumindest in der Hauptbetriebsart, und bevorzugt auch in der Nebenbetriebsart, ein Restgas (26), welches sich bei der Abtrennung des Synthesegases (25) von einem aus Biomasse (5) erzeugten Biogases (15) ergibt, einer Aufbereitung (60, 70) zugeführt wird, um hierüber weiteres Synthesegas (72) zu erzeugen, welches auch der Methanolsynthesevorrichtung (80) zugeführt wird.

3. Verfahren gemäß Anspruch 2, wobei bei der Aufbereitung das Restgas (26) in einem bestimmten Verhältnis einem Oxyfuel-Brenner (60) und einem Reformer (70) zugeführt wird, wobei im Oxyfuel- Brenner (60) eine Oxidation durchgeführt und das oxidierte Gas ebenfalls dem Reformer (70) zugeführt wird und der Reformer (70) mit diesen Gasen eine Verarbeitung, nämlich insbesondere eine Reduktion, durchführt, um sie als ein weiteres Synthesegas (72) der Methanolsynthesevorrichtung (80) zuführen zu können.

4. Verfahren gemäß einem der Ansprüche 1 bis 3, wobei abhängig von dem verfügbaren grünen Strom oder dem verfügbaren Wasserstoff (45) zwischen der Haupt- und Nebenbetriebsart umgeschaltet wird, oder
Mischformen der Betriebsarten zum Einsatz kommen, wobei die Mischformen sich in dem Verhältnis des Restgases (26) unterscheiden, das entweder dem Generator (50) oder der Aufbereitung (60, 70) zugeführt wird.

5. Verfahren gemäß einem der vorangegangenen Ansprüche, bei dem der Restgasstrom (26) in zwei Teilströme (52, 71) aufgeteilt werden, die an den Generator (50) oder eine Aufbereitung (60, 70) geleitet werden.

6. Verfahren gemäß Anspruch 4 oder 5, wobei die Umschaltung oder Aufteilung automatisiert in Abhängigkeit von dem für die Elektrolyse verfügbaren Stroms oder der Menge des Wasserstoffs (45) der Methanolsynthesevorrichtung (80) stattfindet.

7. Verfahren gemäß einem der vorangegangenen Ansprüche, bei dem in der Hauptbetriebsart und Nebenbetriebsart aus Biomasse (5) ein Biogas (15) erzeugt wird und in einem Hydrogenabtrenner (20) aus dem Biogas (15) das Synthesegas (25) abgetrennt wird, welches Wasserstoff H₂ und insbesondere auch Kohlendioxid CO₂ umfasst.

8. Verfahren gemäß einem der vorangegangenen Ansprüche, bei dem Prozesswärme, die von dem Generator (50) und/oder dem Oxyfuel-Brenner (60) erzeugt wird, an einen HyGas-Erzeuger (10) zur Erzeugung des Biogases (15) und/oder an einen Reformer (70) geleitet wird.

9. Verfahren gemäß einem der vorangegangenen Ansprüche, mit einem Speicher (62) für Sauerstoff, wobei in der Hauptbetriebsart der Speicher (62) mit elektrolytisch gewonnenem Sauerstoff befüllt wird
und der Sauerstoff in der Nebenbetriebsart zum Betrieb des Oxyfuel-Brenners (60) verwendet wird.

10. Verfahren gemäß einem der vorangegangenen Ansprüche, mit einem Speicher (63) für CO2, wobei in der Nebenbetriebsart der Speicher (63) mit aus dem Synthesegas (25) gewonnenem CO2 befüllt werden kann und das gespeicherte CO2 in der Hauptbetriebsart zur Methanolsynthese genutzt werden kann.

11. Verfahren gemäß einem der vorangegangenen Ansprüche, wobei ein Teil des grünen Stromes dazu eingesetzt wird, um mithilfe einer Abscheidevorrichtung (64) zusätzliches CO2 durch Abscheidung aus der Umgebungsluft (Direct Air Capture -DAC) zu erzeugen und dem Synthesegas (25) hinzuzufügen.

12. System zur Herstellung von Methanol, mit einem HyGas-Erzeuger (10) zur Speisung eines Hydrogenabtrenners (20), der eingerichtet ist, vor allem Wasserstoff H₂ abzuscheiden, welches als Synthesegas (25) einer Methanolsynthesevorrichtung (80) zuführbar ist, wobei verbleibende Anteile aus dem Hydrogenabtrenner (20) als ein Restgas (26) einem Oxyfuel-Brenner (60) und/oder einem Reformer (70) und/oder einem Generator (50) zugeführt werden können, wobei
das System eingerichtet ist, in einer Hauptbetriebsart Elektrolyse-Wasserstoff (45) der Methanolsynthesevorrichtung (80) zuzuführen,
und
eingerichtet ist, in eine Nebenbetriebsart umzuschalten, bei der kein Elektrolyse-Wasserstoff (45) zugeführt wird,
**dadurch gekennzeichnet, dass**
eine Regelung (30) vorgesehen ist, welche konfiguriert ist, um bei einer Reduktion des der Methanolsynthesevorrichtung (80) zugeführten Wasserstoffs, insbesondere des Elektrolysewasserstoffs (45), einen Restgasstrom (26, 52) von dem Hydrogenabtrenner (20) an einen Generator (50) zu starten oder zu erhöhen, indem insbesondere ein Restgasstrom (26, 72) von dem Hydrogenabtrenner (20) an den Reformer (70) und bevorzugt auch den Oxyfuelbrenner (60) entsprechend reduziert wird.

## Claims

1. Process for producing methanol,
in which a synthesis gas (25) that has been recovered from biomass (5) is fed to a methanol synthesis apparatus (80),
wherein
in a main operating mode in which sufficient electrical power is available for electrolytic hydrogen recovery, correspondingly electrolytically recovered hydrogen (45) is fed to the methanol synthesis apparatus (80),
and wherein, in a secondary operating mode in which insufficient electrical power is available for electrolytic production of hydrogen (45),
a tail gas (26) that arises from a biogas (15) recovered from the biomass (5) on removal of the synthesis gas (25) is fed to a generator (50) in order to provide electrical power for apparatuses (10, 20, 30, 40, 70, 80) involved in the process.

2. Process according to Claim 1, wherein, at least in the main operating mode, and preferably also in the secondary operating mode, a tail gas (26) that arises from a biogas (15) produced from biomass (5) on removal of the synthesis gas (25) is fed to a processing operation (60, 70) in order thereby to produce further synthesis gas (72) which is also fed to the methanol synthesis apparatus (80).

3. Process according to Claim 2, wherein, in the processing operation, the tail gas (26) is fed in a particular ratio to an oxyfuel burner (60) and a reformer (70), wherein an oxidation is conducted in the oxyfuel burner (60) and the oxidized gas is likewise fed to the reformer (70), and the reformer (70) conducts a processing operation with these gases, namely a reduction in particular, in order to be able to supply it as a further synthesis gas (72) to the methanol synthesis apparatus (80).

4. Process according to any of Claims 1 to 3, wherein the system switches between the main operating mode and secondary operating mode depending on the available green power or the available hydrogen (45), or
mixed forms of the operating modes are used, wherein the mixed forms differ in the ratio of the tail gas (26) that is fed either to the generator (50) or to the processing operation (60, 70).

5. Process according to any of the preceding claims, in which the tail gas stream (26) is divided into two substreams (52, 71) that are passed to the generator (50) or a processing operation (60, 70).

6. Process according to Claim 4 or 5, wherein the switchover or division takes place in an automated manner depending on the current available for the electrolysis or the amount of hydrogen (45) from the methanol synthesis apparatus (80).

7. Process according to any of the preceding claims, in which, in the main operating mode and secondary operating mode, a biogas (15) is produced from biomass (5) and the synthesis gas (25) is separated from the biogas (15) in a hydrogen separator (20) and comprises hydrogen H2 and in particular also carbon dioxide CO2.

8. Process according to any of the preceding claims, in which process heat which is generated by the generator (50) and/or the oxyfuel burner (60) is passed to a HyGas generator (10) for generation of the biogas (15) and/or to a reformer (70).

9. Process according to any of the preceding claims, having a storage means (62) for oxygen, wherein, in the main operating mode, the storage means (62) is filled with electrolytically recovered oxygen,
and the oxygen is used in the secondary operating mode for operation of the oxyfuel burner (60).

10. Process according to any of the preceding claims, having a storage means (63) for CO2, wherein, in the secondary operating mode, the storage means (63) can be filled with CO2 recovered from the synthesis gas (25), and the stored CO2 can be utilized in the main operating mode for methanol synthesis.

11. Process according to any of the preceding claims, wherein a portion of the green power is used to produce additional CO2 by separation from ambient air (direct air capture - DAC) by means of a separation apparatus (64) and to add it to the synthesis gas (25).

12. System for production of methanol, having a HyGas generator (10) for feeding a hydrogen separator (20) set up to separate hydrogen H2 in particular, which is feedable as synthesis gas (25) to a methanol synthesis apparatus (80), wherein remaining fractions from the hydrogen separator (20) may be fed as a tail gas (26) to an oxyfuel burner (60) and/or a reformer (70) and/or a generator (50), wherein
the system is set up, in a main operating mode, to feed electrolysis hydrogen (45) to the methanol synthesis apparatus (80),
and
is set up to switch to a secondary operating mode in which no electrolysis hydrogen (45) is supplied,
**characterized in that**
a controller (30) is provided, which is configured to start or to increase a tail gas stream (26, 52) from the hydrogen separator (20) to a generator (50) in the event of a reduction in the hydrogen fed to the methanol synthesis apparatus (80), especially the electrolysis hydrogen (45), especially by correspondingly reducing a tail gas stream (26, 72) from the hydrogen separator (20) to the reformer (70) and preferably also the oxyfuel burner (60).

## Revendications

1. Procédé de préparation de méthanol,
dans lequel un gaz de synthèse (25), qui a été obtenu à partir de biomasse (5), est amené à un dispositif de synthèse de méthanol (80),
où
dans un mode de fonctionnement principal, dans lequel un courant électrique suffisant pour une production électrolytique d'hydrogène est disponible, de l'hydrogène obtenu électrolytiquement (45) est en conséquence amené au dispositif de synthèse de méthanol (80),
et où, dans un mode de fonctionnement secondaire, dans lequel aucun courant électrique suffisant pour la production électrolytique d'hydrogène (45) n'est disponible,
un gaz résiduel (26), qui résulte de la séparation du gaz de synthèse (25) d'un biogaz (15) obtenu à partir de la biomasse (5), est amené à un générateur (50) afin de fournir du courant électrique pour des dispositifs (10, 20, 30, 40, 70, 80) participant au procédé.

2. Procédé selon la revendication 1, dans lequel, au moins dans le mode de fonctionnement principal, et de préférence également dans le mode de fonctionnement secondaire, un gaz résiduel (26), qui résulte de la séparation du gaz de synthèse (25) d'un biogaz (15) produit à partir de biomasse (5), est amené à un traitement (60, 70) afin de produire, par ce biais, un gaz de synthèse supplémentaire (72), lequel est également amené au dispositif de synthèse de méthanol (80).

3. Procédé selon la revendication 2, dans lequel, lors du traitement, le gaz résiduel (26) est amené dans un rapport déterminé à un brûleur d'oxycombustible (60) et à un reformeur (70), dans lequel, dans le brûleur d'oxycombustible (60), une oxydation est effectuée et le gaz oxydé est également amené au reformeur (70), et le reformeur (70) effectue, avec ces gaz, un traitement, à savoir notamment une réduction, afin de pouvoir les amener, en tant que gaz de synthèse supplémentaire (72), au dispositif de synthèse de méthanol (80).

4. Procédé selon l'une quelconque des revendications 1 à 3, dans lequel, en fonction du courant vert disponible ou de l'hydrogène disponible (45), il est procédé à une commutation entre le mode de fonctionnement principal et le mode de fonctionnement secondaire, ou
des formes mixtes des modes de fonctionnement sont mises en œuvre, les formes mixtes se distinguant par le rapport du gaz résiduel (26) qui est amené soit au générateur (50), soit au traitement (60, 70).

5. Procédé selon l'une quelconque des revendications précédentes, dans lequel le flux de gaz résiduel (26) est divisé en deux flux partiels (52, 71), qui sont dirigés vers le générateur (50) ou vers un traitement (60, 70).

6. Procédé selon la revendication 4 ou 5, dans lequel la commutation ou la répartition s'effectue de manière automatisée en fonction du courant disponible pour l'électrolyse ou de la quantité d'hydrogène (45) du dispositif de synthèse de méthanol (80).

7. Procédé selon l'une quelconque des revendications précédentes, dans lequel, dans le mode de fonctionnement principal et dans le mode de fonctionnement secondaire, un biogaz (15) est produit à partir de biomasse (5) et, dans un séparateur d'hydrogène (20), le gaz de synthèse (25) est séparé du biogaz (15), lequel comprend de l'hydrogène H2 et en particulier également du dioxyde de carbone CO2.

8. Procédé selon l'une quelconque des revendications précédentes, dans lequel une chaleur de procédé, qui est générée par le générateur (50) et/ou par le brûleur d'oxycombustible (60), est conduite vers un générateur HyGas (10) pour la production du biogaz (15) et/ou vers un reformeur (70).

9. Procédé selon l'une quelconque des revendications précédentes, comprenant un réservoir (62) pour l'oxygène, dans lequel, dans le mode de fonctionnement principal, le réservoir (62) est rempli avec de l'oxygène obtenu par électrolyse.
et l'oxygène est utilisé, dans le mode de fonctionnement secondaire, pour faire fonctionner le brûleur d'oxycombustible (60).

10. Procédé selon l'une quelconque des revendications précédentes, comprenant un réservoir (63) pour le CO2, dans lequel, dans mode de fonctionnement secondaire, le réservoir (63) peut être rempli avec du CO2 obtenu à partir du gaz de synthèse (25) et le CO2 stocké peut être utilisé, dans le mode de fonctionnement principal, pour la synthèse de méthanol.

11. Procédé selon l'une quelconque des revendications précédentes, dans lequel une partie du courant vert est utilisée afin de produire, au moyen d'un dispositif de captage (64), du CO2 supplémentaire par captage dans l'air ambiant (Direct Air Capture - DAC) et de l'ajouter au gaz de synthèse (25).

12. Système pour la fabrication de méthanol, avec un générateur HyGas (10) pour l'alimentation d'un séparateur d'hydrogène (20), qui est adapté pour séparer principalement de l'hydrogène H2, lequel peut être amené, en tant que gaz de synthèse (25), à un dispositif de synthèse de méthanol (80), les fractions restantes provenant du séparateur d'hydrogène (20) pouvant être amenées, en tant que gaz résiduel (26), à un brûleur d'oxycombustible (60) et/ou à un reformeur (70) et/ou à un générateur (50), dans lequel
le système est adapté pour amener, dans le mode de fonctionnement principal, de l'hydrogène d'électrolyse (45) au dispositif de synthèse de méthanol (80),
et
est adapté pour commuter vers un mode de fonctionnement secondaire, dans lequel aucun hydrogène d'électrolyse (45) n'est amené,
**caractérisé en ce que**
un dispositif de régulation (30) est prévu, lequel est configuré pour, lors d'une réduction de l'hydrogène amené au dispositif de synthèse de méthanol (80), en particulier de l'hydrogène d'électrolyse (45), démarrer ou augmenter un flux de gaz résiduel (26, 52) du séparateur d'hydrogène (20) vers un générateur (50), en réduisant en particulier un flux de gaz résiduel (26, 72) du séparateur d'hydrogène (20) vers le reformeur (70) et de préférence également vers le brûleur d'oxycombustible (60) en conséquence.
